# EUROPÄISCHE PATENTSCHRIFT

(11) **EP 2 599 770 B1**
(45) Veröffentlichungstag und Bekanntmachung des Hinweises auf die Patenterteilung: **10.08.2016**
(21) Anmeldenummer: 12194859.0
(22) Anmeldetag: 29.11.2012
(51) Int. Cl.: C07C 263/10

(54) **Verfahren zur Herstellung von Isocyanaten**
Method for producing isocyanates
Procédé destiné à la fabrication d'isocyanates

(30) Priorität: 02.12.2011 DE 102011087654
(43) Veröffentlichungstag der Anmeldung: 05.06.2013
(73) Patentinhaber: Covestro Deutschland AG, 51373 Leverkusen (DE)
(72) Erfinder: Kintrup, Jürgen, 51373 Leverkusen (DE); Lehner, Peter, Baytown, TX Texas 77523-0000 (US); Jakobs, Eric, 47829 Krefeld (DE); Soppe, Alfred, 201507 Caojing (CN); Werner, Knud, 47800 Krefeld (DE)
(74) Vertreter: Levpat

(56) Entgegenhaltungen:
- WO-A1-2007/131700
- WO-A1-2007/134774
- WO-A1-2008/131870

## Beschreibung

Die Erfindung betrifft ein Verfahren zur Herstellung von Isocyanaten in einem Anlagenverbund umfassend eine Isocyanat-Produktionsanlage, eine Chlor-Produktionsanlage und eine Phosgen-Produktionsanlage, bei dem als Nebenprodukt gebildetes Kohlenstoffdioxid teilweise bis vollständig mit dem in der Chlor-Produktionsanlage gebildeten gasförmigen Chlor auskondensiert wird, in den Phosgen-Herstellungsprozess gelangt und dadurch nach der Phosgenherstellung der überwiegende Teil des gebildeten Kohlenstoffdioxids gasförmig aus dem Anlagenverbund ausgeschleust wird.

Bei der Herstellung von Isocyanaten durch Phosgenierung der korrespondierenden primären Amine entsteht als Nebenprodukt Chlorwasserstoffgas. Das Chlorwasserstoffgas ist üblicherweise mit gasförmigen Substanzen aus der Phosgensynthese oder anderen Verfahrensstufen, wie z. B. Kohlenstoffoxiden (CO und CO₂) und Phosgen, verunreinigt. Es kann für die Herstellung von Isocyanaten wiederverwendet werden, indem man aus ihm Chlor herstellt, das für die Phosgensynthese benötigt wird.

Die Herstellung von Chlor aus Chlorwasserstoff ist im Stand der Technik hinlänglich bekannt. Es gibt verschiedene elektrolytische Verfahren zur Chlorherstellung, die von Salzsäure (HCl (aq)) oder Akalimetallchloriden ausgehen. Daneben gewinnt die direkte katalytische Oxidation von gasförmigem Chlorwasserstoff (HCl (g)),

4 HCl (g) + O₂ (g) → 2 Cl₂ (g) + 2 H₂O (g),

im Folgenden kurz Deacon-Verfahren genannt, zunehmend an Bedeutung.

An den Eduktgasstrom einer Deacon-Anlage sind bestimmte Reinheitsanforderungen zu stellen. So ist aus DE 10 2006 024 549 A1 bekannt, dass der CO-Gehalt im Eintrittsstrom weniger als 1 Vol.-%, insbesondere weniger als 0,5 Vol.-%, bezogen auf das Gesamtvolumen des Eintrittsstroms, betragen sollte. Ein höherer CO-Gehalt kann infolge beschleunigter Katalysatordesaktivierung die Anlagenverfügbarkeit beeinträchtigen. Weiterhin ist bekannt, dem Eintrittsstrom neben den Edukten Chlorwasserstoff und Sauerstoff noch Wasser zuzufügen, um die Temperaturverteilung innerhalb der Katalysatorschicht zu glätten **(**JP 2001 019 405 A**).** Der positive Einfluss von Wasser macht sich auch insofern bemerkbar, als er den negativen Einfluss von CO zumindest teilweise kompensieren kann. Indem die Geschwindigkeit der stark exothermen CO-Oxidation verlangsamt wird, mindert die Dosierung von Wasser bevorzugt die Ausbildung lokaler Hotspots, in denen besonders bevorzugt ein für die Aktivität negativer Sinterungsprozess beschleunigt abläuft und in denen bevorzugt die Aktivkomponente des Katalysators (z. B. Ruthenium) thermisch ausgetrieben wird. Weiterhin mindert die Dosierung von Wasser die Ausbildung flüchtiger Metallcarbonyl- und Metallchlorocarbonyl-Verbindungen aus dem eingesetzten Katalysator, deren Ausbildung auch schon bei Temperaturen unterhalb von 350 °C einen beschleunigten Austrag von katalytisch aktivem Metall (z. B. Ruthenium) begünstigt.

Im Sinne eines geschlossenen Produktionsverbundes ist es vorteilhaft, eine Anlage zur Produktion von Chlor aus Chlorwasserstoff mit einer Isocyanat-Produktionsanlage zu koppeln, um so durch die maximale Ausnutzung von Kreislaufströmen den apparativen Aufwand und den Energieverbrauch und somit die Kosten zu minimieren. Besonders vorteilhaft ist dabei die Chlorproduktion über ein Deacon-Verfahren.

In diesem Zusammenhang können jedoch bestimmte Verunreinigungen, die in einem aus einer Isocyanatproduktion stammenden Chlorwasserstoffstrom enthalten sind, problematisch sein. Hier ist insbesondere das Kohlendioxid zu nennen, welches sich, wenn keine besonderen Maßnahmen getroffen würden, in den Kreislaufströmen immer mehr anreichern würde.

Daher ist es üblich, Kohlendioxid über einen geeigneten Purge-Strom (Abgas-Strom) auszuschleusen. In einer kombinierten Deacon-/Isocyanat-Produktionsanlage geschieht dies nach dem Stand der Technik so, dass der im Deacon-Prozess erhaltene chlorreiche Produktstrom nach Trocknung und Kompression in eine Destillationskolonne eingeleitet wird. Dabei wird der Kopfkondensator der Destillationskolonne zur Chlorkondensation genutzt und der Sumpfverdampfer zum Austreiben von CO₂, nicht umgesetztem Sauerstoff und ggf. anderen flüchtigen Komponenten verwendet. Dieser Stand der Technik ist beispielsweise bekannt aus EP 0 329 385 A2 oder DE 10 2006 023 581 A1**.** Das abgetrennte Chlor kann nun in den Isocyanatprozess geführt und zur Phosgensynthese verwendet werden.

Der in der Chlor-Destillation ausgetriebene Gasstrom enthält den nicht umgesetzten Sauerstoff (Sauerstoff wird im Deacon-Prozess üblicherweise in großem Überschuss eingesetzt), das CO₂ sowie weitere gasförmige Substanzen und wird, erforderlichenfalls nach einer Waschstufe, in die Deacon-Reaktion rückgeführt. Durch die Rückführung des Restgases wird ein Kreislauf aufgebaut, und es kommt zu einer Anreicherung von Gasen wie z. B. CO₂, die ausgeschleust werden müssen.

Dazu wird ein Teil des in die Deacon-Reaktion zurückgeführten, sauerstoffhaltigen Restgases aus dem Kreislauf ausgeschleust und so die Anreicherung solcher gasförmiger Komponenten, die in den folgenden Verfahrensstufen nicht (wie Sauerstoff) weiterreagieren, im Kreislaufstrom verhindert. Die Ausschleusung muss vor Abgabe in die Umgebung behandelt werden, da sie noch restliches Chlor enthält.

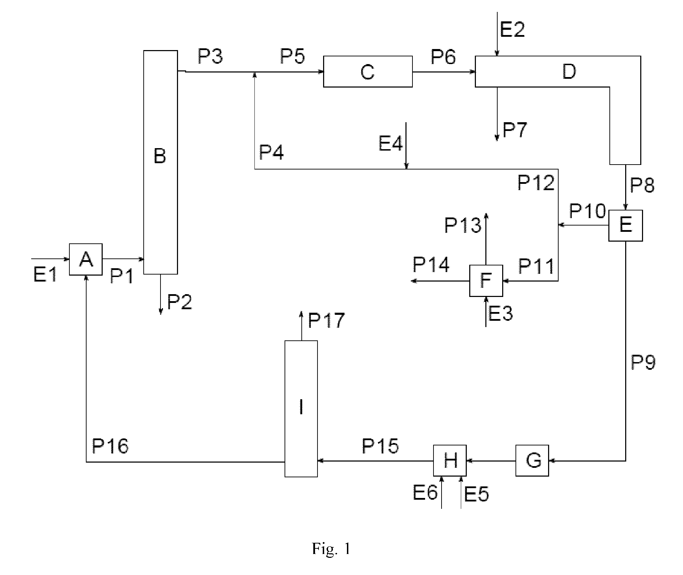

WO 2007/131700 A1 offenbart einen kombinierten Prozess zur Herstellung von Isocyanaten und Chlor, in welchem das in der Umsetzung eines primären Amins mit Phosgen (Stufe 2 in Fig. 1) gebildete Koppelprodukt Chlorwasserstoff vom gebildeten Isocyanat abgetrennt (Stufe 3 in Fig. 1) und anschließend mit Sauerstoff katalytisch zu Chlor oxidiert wird (Stufe 4 in Fig. 1). Das so gebildete Chlor wird abgekühlt (Stufe 5 in Fig. 1), getrocknet (Stufe 6 in Fig. 1) und nach einer nicht näher beschriebenen Abtrennung von Restströmen wie Sauerstoff sowie ggf. Stickstoff und Kohlendioxid (Stufe 7 in Fig. 1) zur Herstellung des in der Isocyanatproduktion eingesetzten Phosgens (Stufe 1 in Fig. 1) verwendet. Im Unterschied zur vorliegenden Erfindung wird Kohlendioxid hauptsächlich in Stufe 7, also unmittelbar vor der Rezyklierung des gebildeten Chlors in die Phosgensynthese, aus dem Prozess entfernt.

WO 2008/131870 A1 offenbart ebenfalls einen kombinierten Prozess zur Herstellung von Isocyanaten und Chlor (Patentanspruch 17). In der Isocyanatsynthese (Schritt b)) wird als Koppelprodukt ein Gas enthaltend Chlorwasserstoff und Kohlenmonoxid erhalten, welches nach Abtrennung des Isocyanats (Schritt c)) und Reinigung (Schritt d)) einer Oxidation (Schritte e) bis g)) unterworfen wird. In dieser Oxidation wird zunächst Kohlenmonoxid zu Kohlendioxid (Schritt e)) und dann Chlorwasserstoff zu Chlor (Schritt g)) oxidiert. Das gebildete Chlor wird vom gebildeten Kohlendioxid abgetrennt (Schritt h)) und optional in die Phosgensynthese (Schritt a)) zurückgeführt (Schritt i)). Auch hier erfolgt also die Trennung von Kohlendioxid und Chlor (Schritt h)) unmittelbar vor der Rezyklierung des gebildeten Chlors in die Phosgensynthese.

Auch WO 2007/134774 A1 offenbart einen kombinierten Prozess zur Herstellung von Isocyanaten und Chlor (Patentanspruch 1). In der Isocyanatsynthese (Schritt b)) wird als Koppelprodukt ein Gas enthaltend Chlorwasserstoff und Kohlenmonoxid erhalten, welches nach Abtrennung des Isocyanats (Schritt c)) mit Chlor versetzt wird, um das vorhandene Kohlenmonoxid zu Phosgen umzusetzen (Schritt d)). Das so gebildete Phosgen wird abgetrennt (Schritt e)) und optional in die Isocyanatsynthese (Schritt b)) zurückgeführt (Schritt f)). Der an Kohlenmonoxid abgereicherte Chlorwasserstoff-Gasstrom wird optional zu Chlor oxidiert (Schritt g)). Evtl. noch vorhandenes Kohlendioxid wird nach dieser Oxidation in einer Trennstufe ausgeschleust (siehe Zeichnungen). Auch hier erfolgt also die Trennung von Chlor und Kohlendioxid vor der Rezyklierung des gebildeten Chlors in die Phosgensynthese.

Der Stand der Technik zur Entfernung von Chlor aus Abgasen ist eine Wäsche mit Natronlauge (siehe bspw. Ullmann's Encyclopedia of Industrial Chemistry 2006, Chapter Chlorine, insbesondere S. 80, Treatment of Gaseous Effluents), bei der das Chlor in der Waschflüssigkeit absorbiert und chemisch umgesetzt wird:

Cl₂ + 2 NaOH → NaCl + NaOCl + H₂O

Um das Chlor sicher zu entfernen, wird die Wäsche mit einem NaOH-Überschuss betrieben. Aufgrund dieses Überschusses wird jedoch auch alles CO₂ mit entfernt:

CO₂ + 2 NaOH → Na₂CO₃ + H₂O

Diese *unselektive* Chlorabsorption hat daher bei Einsatz in einer kombinierten Deacon/ Isocyanat-Produktionsanlage den Nachteil, dass bei ihr Natronlauge nicht nur zur Chlorabsorption, sondern auch zur an sich unnötigen Absorption von CO₂ verbraucht wird. Ein weiterer Nachteil ist, dass in der Waschflüssigkeit Na₂CO₃ ausfallen und den Wäscher blockieren kann. In einem solchen Fall ist dessen Funktionsfähigkeit nicht mehr gegeben und der Deacon-Prozess muss abgefahren werden.

Eine *selektive* Chlorabsorption aus einem CO₂-haltige Gasstrom ist jedoch auch schon in mehreren Veröffentlichungen behandelt worden. So wird etwa in **US H1417** ein Prozess beschrieben, bei dem in einer Waschkolonne mit einer Mischung aus Natriumthiosulfat (Na₂S₂O₃) und Natronlauge Chlor absorbiert und reduziert wird. Dabei wird Natronlauge so dosiert, dass sich in der Waschflüssigkeit ein pH-Wert von ca. 8,5 einstellt. Bei diesem pH-Wert liegt kein NaOH vor, sondern NaHCO₃. Dies rührt daher, dass so wenig NaOH zugeführt wird, dass nur ein Teil des CO₂ absorbiert wird, NaHCO₃ bildet und dieses dann bei der Reduktion des Chlors verbraucht und das CO₂ wieder freigesetzt wird:

10 CO₂ + 10 NaOH → 10 NaHCO₃

4 Cl₂ + Na₂S₂O₃ + 10 NaHCO₃ → 8 NaCl + 2 Na₂SO₄ + 5 H₂O + 10 CO₂

Ein Nachteil dieser Fahrweise ist die Gefahr der Zersetzung des Natriumthiosulfats, bei der Schwefelausfällungen die Waschkolonne blockieren können. Diese Zersetzung tritt dann auf, wenn z. B. lokal in der Kolonne der pH-Wert der Absorptionslösung zu klein wird. Unter anderem aus diesem Grund wird dieses Verfahren bei Abgasströmen mit höherem Chlorgehalt und-mengenstrom nicht angewendet.

Als alternatives Reduktionsmittel wird in der US H1417 Sulfit angegeben, wobei jedoch darauf hingewiesen wird, dass Sulfit bei Schwankungen im pH-Wert besonders leicht zur Bildung von SO₂ neigt. Dieses SO₂ würde den zu reinigenden Gasstrom kontaminieren.

Zusätzlich ist die Verwendung eines Reduktionsmittels, sei es Thiosulfat oder Sulfit, teuer.

In der DE 24 13 358 A1 wird eine mehrstufige Absorption von Chlor mit Natronlauge vorgeschlagen, wobei frische Natronlauge im Gegenstrom zum chlor- und CO₂-haltige Gasstrom geführt wird. Die Natronlauge wird so dosiert, dass sie sich mit dem CO₂ komplett zu NaHCO₃ umsetzt, das dann wieder mit Chlor reagiert und dabei das vorher gebundene CO₂ abgibt:

CO₂ + 2 NaOH → 2 NaHCO₃

Cl₂ + 2 NaHCO₃ → NaCl + NaOCl + H₂O + 2 CO₂

Ein Nachteil dieses Verfahrens ist die in DE 24 13 358 A1 angesprochene schwierige Prozessführung zur vollständigen Chlorabsorption. Weiterhin besteht bei einem niedrigen pH-Wert die Gefahr, dass das Hypochlorit zum Chlorat weiterreagiert. Chlorat kann im Vergleich zu Hypochlorit nur sehr schwer zersetzt werden, so dass das entstehende Abwasser ein Entsorgungsproblem bereitet. Da auch kein Laugeüberschuss vorhanden ist, ist die gebildete Hypochloritlösung instabil und kann sich leicht zersetzen.

In WO 2004/037 718 A2 wird auch die Abtrennung von Substanzen wie Phosgen, Lösungsmittelresten, CO₂ etc. aus dem einzusetzenden Chlorwasserstoffgas durch Absorption in Wasser beschrieben. Die dabei entstehende Salzsäure muss anschließend aufwändig in Chlorwasserstoffgas und Wasser zerlegt werden, was erhebliche Kosten verursacht.

Zusammenfassend bleibt daher festzuhalten, dass die unselektive Absorption des Chlors aus dem auszuschleusenden CO₂-haltige Purge-Strom einen erhöhten Bedarf an Natronlauge zur Folge hat, während die aus dem Stand der Technik bekannten Methoden zu selektiven Absorption von Chlor aus einem CO₂-haltige Purge-Strom mit einer Reihe anderer Nachteile behaftet sind. Es bestand somit ein Bedarf an einer Möglichkeit, aus einer kombinierten Deacon-/Isocyanat-Produktionsanlage einen gasförmigen, CO₂-haltige Strom auszuschleusen, ohne dass dabei die oben geschilderten Nachteile auftreten.

Ein Gegenstand der vorliegenden Erfindung ist ein Verfahren zur Herstellung eines Isocyanats umfassend die Schritte
(i) Umsetzung eines ein primäres Amin enthaltenden Stroms **E1** mit einem phosgenhaltigen Strom **P16** in einem Reaktor **A** unter Ausbildung eines Produktstroms **P1**, der das gewünschte Isocyanat, Chlorwasserstoff, nicht umgesetztes Phosgen und Kohlenstoffoxide umfasst,
(ii) Auftrennung des Produktstromes **P1** in einer Trennvorrichtung **B** in einen das Isocyanat umfassenden flüssigen Produktstrom **P2** und einen gasförmigen, Chlorwasserstoff, nicht umgesetztes Phosgen und Kohlenstoffoxide umfassenden Produktstrom **P3,**
(iii) Vermischung des Chlorwasserstoff, nicht umgesetztes Phosgen und Kohlenstoffoxide umfassenden Produktstroms **P3** mit einem gasförmigen, Sauerstoff umfassenden Produktstrom **P4** unter Bildung eines gasförmigen Mischstromes **P5,**
(iv) Oxidation des gasförmigen Mischstromes **P5** an einem Katalysator in einer oxidativen Verfahrensstufe **C** unter Bildung eines gasförmigen Produktstromes **P6,** der Chlorwasserstoff, Kohlenstoffdioxid, insbesondere das Kohlenstoffdioxid aus **P5** sowie durch Oxidation von Kohlenstoffmonoxid und Phosgen entstandenes Kohlenstoffdioxid, überschüssigen Sauerstoff, Chlor und Wasser umfasst,
(v) Teilweise bis vollständige Abtrennung des Chlorwasserstoffs und des Wassers aus **P6** als Salzsäure-haltigen Strom **P7** durch Behandlung von **P6** mit einem Absorptionsmittel **E2,** bevorzugt mit Wasser oder 1 bis 20%iger Salzsäure, bevorzugt 4 bis 18%iger Salzsäure, besonders bevorzugt 4 bis 15%iger Salzsäure, in einer Trennvorrichtung **D** unter Ausbildung eines an Chlorwasserstoff und Wasser abgereicherten Produktstromes **P8,** wobei bevorzugt 70 % bis 100 % des in **P6** enthaltenen Chlorwasserstoffs und 99,00 % bis 100 %, bevorzugt 99,99 % bis 100 %, besonders bevorzugt 100 %, des in **P6** enthaltenen Wassers aus diesem Strom abgetrennt werden,
(vi) Auftrennung des Produktstromes **P8** in einer Trennvorrichtung **E** in einen flüssigen chlorreichen Produktstrom **P9,** der Chlor, und zwar bevorzugt 50 % bis 100 % des Chlors aus **P8,** Kohlenstoffdioxid und Sauerstoff umfasst, und in einen gasförmigen, chlorarmen Produktstrom **P10,** der die Restmenge des nicht in **P9** enthaltenen Chlors aus **P8** sowie Kohlenstoffdioxid und Sauerstoff umfasst, wobei **P9** und/oder **P10** auch noch in Schritt (v) nicht abgetrennten Chlorwasserstoff enthalten können,
(vii) Aufteilung des gasförmigen Produktstromes **P10** in einen gasförmigen Purge-Strom **P11** und in einen gasförmigen Produktstrom **P12,** bevorzugt in einem zeitgemittelten Massenverhältnis **P12 : P11** von 100 : 1 bis 5 : 1, besonders bevorzugt von 50 : 1 bis 10 : 1, wobei die Aufteilung von **P10** kontinuierlich oder intermittierend, d. h. in Intervallen (sobald sich auszuschleusende Gase in größerer Menge angereichert haben) erfolgen kann,
(viii) Behandlung des Purge-Stromes **P11** mit einer wässrigen Base **E3,** bevorzugt mit einer wässrigen Alkalimetall- oder Erdalkalimetallhydroxidlösung, besonders bevorzugt Natronlauge, in einer Abgasbehandlungsstufe **F** unter Bildung eines gasförmigen Purge-Stromes **P13** und eines flüssigen Abwasserstromes **P14,**
(ix) Vermischung des gasförmigen Stromes **P12** mit einem gasförmigen, Sauerstoff umfassenden Strom **E4** unter Ausbildung des gasförmigen, Sauerstoff umfassenden Produktstromes **P4,** welcher in Schritt (iii) eingesetzt wird,
(x) Verdampfung des flüssigen chlorreichen Produktstroms **P9** in einem Verdampfer **G,** Vermischung des so erhaltenen gasförmigen chlorreichen Produktstroms mit Kohlenstoffmonoxid **E5** und Chlor **E6** und Umsetzung des so erhaltenen Gemisches in einem Reaktor **H** zu einem gasförmigen Produktstrom **P15** umfassend Phosgen und Kohlenstoffoxide,
(xi) Auftrennung des gasförmigen Produktstroms **P15** in einer Trennvorrichtung **I** in einen phosgenreichen Produktstrom **P16,** welcher in Schritt (i) eingesetzt wird und Phosgen, und zwar bevorzugt 70 % bis 100 % des Phosgens aus **P15**, und Kohlenstoffoxide umfasst, und in einen phosgenarmen Purge-Strom **P17,** der die Restmenge des nicht in **P16** enthaltenen Phosgens aus **P15** sowie Kohlenstoffoxide umfasst,
wobei
in Schritt (vi) die Auftrennung des Produktstromes **P8** in einen flüssigen chlorreichen Produktstrom **P9** und in einen gasförmigen chlorarmen Produktstrom **P10** durch Abkühlung von **P8** auf eine Temperatur von -10 °C bis -80 °C unter einem absoluten Druck von 1,0 bar bis 30 bar ohne sich anschließende Destillation erfolgt.

Im Rahmen dieser Erfindung bezeichnet der Begriff *Produktstrom* einen Strom, der im Wesentlichen ein in einem Verfahrensschritt erhaltenes Wertprodukt (oder mehrere Wertprodukte) enthält, sei es Endprodukt oder Zwischenprodukt (d. h. Edukt für einen weiteren Verfahrensschritt). Davon unterschieden wird der Begriff *Purge-Strom,* welcher einen Strom bezeichnet, der im Wesentlichen aus dem Gesamtprozess auszuschleusende störende Komponenten wie das erwähnte Kohlenstoffdioxid enthält. Erfindungsgemäß umfasst der Begriff *Kohlenstoffoxide* sowohl Kohlenstoffmonoxid als auch Kohlenstoffdioxid.

Bevorzugte *primären Amine* sind solche ausgewählt aus der Gruppe bestehend aus aliphatischen Aminen (bevorzugt 1,6-Diaminohexan, Methylamin, Ethylamin, Propylamin, Butylamin, Pentylamin und Hexylamin, besonders bevorzugt 1,6-Diaminohexan), cycloaliphatischen Aminen (bevorzugt Cyclohexylamin, Isophorondiamin, 4,4'-Diaminodicyclohexylmethan, 2,4'-Diaminodicyclohexylmethan, 2,2'-Diaminodicyclohexylmethan und Gemische der Diaminodicyclohexylmethanisomeren, besonders bevorzugt Isophorondiamin, 4,4'-Diaminodicyclohexylmethan, 2,4'-Diaminodicyclohexylmethan, 2,2'-Diaminodicyclohexylmethan und Gemische der Diaminodicyclohexylmethanisomeren), araliphatischen Aminen (bevorzugt Benzylamin),
und
aromatischen Aminen (bevorzugt Anilin, Chloranilin, Toluylendiamin, 1,5-Diaminonaphthalin, 4,4' -Diaminodiphenylmethan, 2,4' -Diaminodiphenylmethan, 2,2' -Diaminodiphenylmethan, Gemische der Diaminodiphenylmethanisomeren und Gemische der Diaminodiphenylmethanisomeren und deren höheren Homologen [auch allgemein als z. B. MDA, PMDA, Polymer-MDA oder Di- und Polyamine der Diphenylmethanserie bezeichnet, d. h. Gemische von Di- und Polyaminen, wie sie aus der sauer katalysierten Kondensation von Anilin und Formaldehyd erhalten werden], besonders bevorzugt Toluylendiamin).

Die *Umsetzung* der primären Amine mit Phosgen zu den korrespondierenden Isocyanaten in Schritt (i) kann dabei grundsätzlich nach allen im Stand der Technik bekannten Verfahren erfolgen. Dabei sind Gas- und Flüssigphasenprozesse denkbar. Flüssigphasenprozesse werden in Gegenwart eines unter den Reaktionsbedingungen inerten Lösungsmittels durchgeführt. Bei Gasphasenprozessen wird der Produktstrom nach der Reaktion durch Durchleiten durch ein inertes Lösungsmittel oder Eindüsen eines inerten Lösungsmittels verflüssigt. Beispiele für geeignete Flüssigphasenprozesse sind in US-A 2007/0299279 und DE-A 103 10 888 beschrieben. Beispiele für geeignete Gasphasenprozesse sind in EP 1 449 826 A1 und EP 2 199 277 A1 beschrieben.

Als *Renktoren **A*** eignen sich dabei erfindungsgemäß grundsätzlich alle dem Fachmann für Phosgenierungsreaktionen bekannten Reaktortypen, z. B. Rohrreaktoren. Geeignete Reaktoren sind besipielsweise in DE-A 103 10 888 und US-A 2007/0299279 beschrieben.

Als *Trennvorrichtung **B*** in Schritt (ii) werden bevorzugt dem Fachmann bekannte Vorrichtungen zur Destillation und/oder Absorption eingesetzt. Der in Schritt (ii) erhaltene das Isocyanat enthaltene Strom **P2** wird nach einem der im Stand der Technik üblichen Verfahren aufgearbeitet und so auf die für die gewünschte Folgeanwendung des Isocyanats nötige Reinheit gebracht. Beispiele für die Reinigung von Roh-Isocyanaten werden z. B. in EP 1 371 634 A1, EP 1 078 669 A1, EP 1475 367 A1, EP 1 792 895 A1, WO 2010/095927 A1, WO 2010/149544 A2 und den dort zitierten Literaturstellen beschrieben.

Der in Schritt (ii) erhaltene Produktstrom **P3** enthält typischerweise neben Chlorwasserstoffgas auch noch folgende Bestandteile:
- Kohlenwasserstoffe und Chlorkohlenwasserstoffe bis zu 20 Vol-% in Summe, bevorzugt bis zu 3,0 Vol-% in Summe, besonders bevorzugt bis zu 1,0 Vol-% in Summe, wobei darin bis zu 1,0 Vol.-%, bevorzugt 0,010 Vol.-% bis 0,10 Vol.-% aromatische Kohlenwasserstoffe und aromatische Chlorkohlenwasserstoffe enthalten sind,
- Kohlenstoffoxide, Stickstoff und weitere Inertgase bis 10 Vol-% in Summe, bevorzugt bis zu 5,0 Vol-% in Summe,
- Phosgen bis zu 1,0 Vol-%, bevorzugt bis zu 0,50 Vol-%.

Erfindungsgemäß erfolgt die *Oxidation* des in Schritt (iii) erhaltenen gasförmigen, Chlorwasserstoff enthaltenden Mischstromes **P5** in Schritt (iv) in einem Deacon-Prozess. Die Reaktionstemperatur beträgt dabei bevorzugt 150 °C bis 500 °C, der Reaktionsdruck 1,0 bar (abs.) bis 25 bar (abs.).

Bevorzugt einzusetzende Katalysatoren enthalten Rutheniumverbindungen, die insbesondere auf Trägern aufgebracht sind. Als Trägermaterialen und/oder Bindemittel für den Träger eignen sich besonders beispielsweise Siliziumdioxid, Graphit, Titandioxid mit Rutil- oder Anatas-Struktur, Zinndioxid, Zirkondioxid, Aluminiumoxid oder deren Gemische, bevorzugt Titandioxid, Zirkondioxid, Aluminiumoxid oder deren Gemische, besonders bevorzugt γ- oder δ-Aluminiumoxid oder deren Gemische. Bevorzugtes Bindemittel ist Aluminiumoxid oder Zirkonoxid. Der Anteil an Bindemittel kann bezogen auf den fertigen Katalysator 1 bis 30 Massen-% betragen, bevorzugt 2 bis 25 Massen-% und ganz bevorzugt 5 bis 20 Massen-%. Das Bindemittel erhöht die mechanische Stabilität (Festigkeit) der Katalysatorformkörper.

Die Aktivität der Katalysatoren kann in Strömungsrichtung des zu oxidierenden Gasstromes veränderlich oder gleich sein. Die Reaktion kann in Fest-, Fließ- oder Wirbelbetten in einer oder mehreren Stufen hintereinander durchgeführt werden. Die Reaktionsführung kann isotherm, quasiisotherm oder adiabat mit Zwischenkühlung, diskontinuierlich oder kontinuierlich, bevorzugt kontinuierlich, erfolgen. Die Edukte können bei Verwendung mehrerer Reaktionsstufen vollständig vor dem ersten Reaktor oder verteilt auf jeden Reaktor zugegeben werden. Es kann auch ein Edukt, bevorzugt der Sauerstoff umfassende Produktstrom **P4,** vor dem ersten Reaktor zugegeben werden, und das zweite Edukt, bevorzugt der das Chlorwasserstoffgas umfassende Produktstrom **P3,** verteilt auf jeden Reaktor zugeführt werden. Der Sauerstoff wird zweckmäßigerweise im Überschuss zum Chlorwasserstoff-Gas eingesetzt, wobei Überschüsse von 100 % der Stöchiometrie und mehr einsetzbar sind. Bevorzugt sind Überschüsse an Sauerstoff zwischen 100 % und 300 % der Stöchiometrie. Die im Chlorwasserstoff umfassenden Strom **P3** enthaltenen Verunreinigungen wie CO und Phosgen werden in Schritt (iv) ebenfalls oxidiert und bilden CO₂ Bevorzugt erfolgt diese Oxidation vollständig, sodass der Strom **P6** keine signifikanten Mengen an Kohlenstoffmonoxid und Phosgen mehr enthält. Nach der Reaktion liegt daher bevorzugt eine Gasmischung **P6** vor, in der neben Chlor, Wasser und dem Überschuss-Sauerstoff nur noch unreagiertes Chlorwasserstoffgas und Kohlenstoffdioxid enthalten sind.

In dem sich anschließenden Schritt (v) wird zunächst das Chlorwasserstoffgas zusammen mit dem größten Teil des Reaktionswassers abgeschieden. Dies geschieht bevorzugt in einem Quenchprozess oder einem Absorptionsschritt oder einer Kombination aus beiden. Die Temperatur beträgt dabei bevorzugt 15 °C bis 150 °C, der Druck beträgt bevorzugt 1,0 bar (abs.) bis 25 bar (abs.) und ist insbesondere bevorzugt der gleiche wie im Reaktionsschritt (iv). Als *Trennvorrichtung **D*** wird bevorzugt eine mehrstufige Vorrichtung eingesetzt, die aus einer Quenche und darauf folgend mindestens einem Absorber besteht. Als Absorptionsmittel **E2** wird bevorzugt Wasser oder verdünnte Salzsäure eingesetzt, wobei als Nebenprodukt konzentrierte Salzsäure entsteht. Es ist auch möglich, andere Absorptionsmittel einzusetzen. In Frage kommen bevorzugt organische Substanzen wie tertiäre Amine oder Heteroaromaten oder auch ionische Flüssigkeiten. Anschließend wird das Reaktionswasser in einem Trocknungsschritt nahezu komplett entfernt. Dazu wird bevorzugt eine mit konzentrierter Schwefelsäure beaufschlagte Kolonne verwendet. Es können auch andere, feste Trocknungsmittel eingesetzt werden, bevorzugt Zeolithe oder auch Aluminiumoxide.

Bei der in Schritt (vi) eingesetzten *Trennvorrichtung **E*** handelt es sich z. B. um eine Vorrichtung zur Kondensation von Gasen, jedoch nicht um eine Destillationsapparatur. Grundsätzlich können alle im Stand der Technik üblichen Kondensatoren eingesetzt werden. Bevorzugt liegt die Kondensationstemperatur je nach Druck in einem Bereich von -80 C bis 0 °C. Der so erhaltene, flüssige chlorreiche Produktstrom **P9** enthält bevorzugt 70 Mol-% bis 100 Mol-% Chlor, 0 Mol-% bis 2,0 Mol-% Sauerstoff, 1,0 Mol-% bis 30 Mol-% Kohlenstoffdioxid, sowie in Summe 0 Mol-% bis 20 Mol-% einen oder mehrere der Stoffe der Gruppe Edelgase, Inerte, Kohlenstoffmonoxid und Chlorwasserstoff.

Bei der in Schritt (vii) vorgenommenen *Aufteilung des Produktstromes **P10*** wird dessen Zusammensetzung nicht verändert; es handelt sich bei diesem Schritt lediglich um eine Teilung des Stroms **P10** in zwei Teilströme **P11** und **P12,** deren Zusammensetzung jeweils die gleiche ist wie die von **P10.** Im *zeitgemittelten Massenverhältnis* bedeutet dabei, dass **P12** und **P11** gemittelt über einen Produktionszyklus bevorzugt im Verhältnis zwischen 100 : 1 und 5 : 1, besonders bevorzugt von 50 : 1 bis 10 : 1, aufgeteilt werden. Dies ist bei intermittierender Ausschleusung des Purge-Stromes **P11** zu beachten.

Bei der in Schritt (xiii) eingesetzten *Abgnsbehnndlungsstufe **F*** handelt es sich bevorzugt um eine Absorptionskolonne, in der das im Teilstrom **P11** enthaltene Chlor und der Chlorwasserstoff mit Natronlauge ausgewaschen werden.

Der in Schritt (ix) zugeführte *Sauerstoff umfassende Strom **E4*** kann ein beliebiger Gasstrom sein, der Sauerstoff enthält, und zwar bevorzugt mindestens 80 Massen-% Sauerstoff, bezogen auf die Gesamtmasse von **E4,** und dessen übrige Bestandteile die nachfolgenden Verfahrensschritte nicht stören (beispielsweise kann **E4** neben Sauerstoff Edelgase oder Stickstoff enthalten). Bevorzugt wird Sauerstoff in einer Reinheit von mindestens 90 % als Strom **E4** eingesetzt.

In Schritt (x) können als *Verdampfen **G*** grundsätzlich alle aus dem Stand der Technik bekannten Verdampfertypen eingesetzt werden. Bevorzugt sind Verdampfer mit senkrecht stehendem Rohrbündel oder senkrecht stehenden Bajonettrohren. Beispiele für geeignete Verdampfer finden sich in Design and Operation of Chlorine Vaporisers (Euro Chlor Publication GEST 75/47, Draft 9th Edition, October 1999, S. 1 bis 49). Als *Reaktor **H*** für die Phosgenherstellung wird bevorzugt ein Rohrbündelreaktor verwendet. Beispiele für geeignete Reaktoren finden sich in Ullmann's Encyclopedia of Industrial Chemistry, DOI 10.1002/14356007.a19_411, Wiley-VCH 2005, Chapter Phosgene, insbesondere S. 3 (Process Description). Zur Phosgenherstellung wird dem gasförmigen chlorreichen Produktstrom neben Kohlenstoffmonoxid frisches Chlor **E6** zugeführt, um vorherige Chlorverluste (bspw. durch unvollständigen Umsatz von Chlorwasserstoff in oxidativen Verfahrensstufe **C)** auszugleichen, und zwar bevorzugt 5 % bis 50 % des über den Verdampfer **G** zugeführten Chlorstroms. Kohlenstoffmonoxid wird bevorzugt im Überschuss eingesetzt, und zwar bevorzugt in einem Überschuss von 1,0 % bis 20 % der Theorie (siehe z. B. EP 0 003 530 A1 und EP 0 134 506 A2).

Bei der in Schritt (xi) eingesetzten *Trennvorrichtung **I*** handelt es sich bevorzugt um einen Kondensator oder einen Destillationsapparat gefolgt von einem Absorber (siehe z. B. Ullmann's Encyclopedia of Industrial Chemistry, DOI 10.1002/14356007.a19_411, Wiley-VCH 2005, Chapter Phosgene, insbesondere S. 3 Figur 1); besonders bevorzugt wird ein Kondensator mit darauf folgendem Absorber eingesetzt. Dort wird das entstandene Phosgen durch Verflüssigung und Absorption von Kohlenstoffdioxid und überschüssigem Kohlenstoffmonoxid sowie ggf. sonstigen vorhandenen inerten Gasen abgetrennt. Ein Teil des Kohlenstoffdioxids (bevorzugt zwischen 10 % und 70 % des in **P15** enthaltenen Kohlenstoffdioxids) wird dabei mit kondensiert. Die verbleibende, Kohlenstoffmonoxid und den restlichen Teil des Kohlenstoffdioxids enthaltende Gasphase wird in eine Abgasbehandlungsstufe geleitet und gelangt von dort ins Freie, so dass auf diesem Weg Kohlenstoffdioxid aus dem Anlagenverbund ausgeschleust wird. In einer bevorzugten Ausführungsform werden in Schritt (xi) 35 % bis 99 %, bevorzugt 40 % bis 95 %, des insgesamt auszuschleusenden Kohlenstoffdioxids über den Purge-Strom **P17** ausgeschleust.

Durch die erfindungsgemäße Vorgehensweise in Schritt (vi) wird demnach der Hauptteil des Kohlenstoffdioxids nicht über eine Ausschleusung aus dem sauerstoffhaltigen, in die Deacon-Reaktion rückgeführten Restgas abgetrennt, wie im Stand der Technik bisher üblich. Vielmehr wird das Kohlenstoffdioxid zum größten Teil zusammen mit dem Chlor durch Kondensation abgetrennt. Es kann sich daher nicht im rückgeführten Gasstrom **P10** aufkonzentrieren. Die Ausschleusung eines Teilstroms **P11** aus diesem Gasstrom ist dann nur noch zur Entfernung anderer Komponenten wie z. B. Stickstoff oder Argon erforderlich. Der Strom **P11** enthält neben Resten von Chlor nur noch einen geringen Anteil an Kohlenstoffdioxid, der aber nicht mehr ins Gewicht fällt, da durch diesen nur unwesentlich mehr wässrige Base **E3** verbraucht wird.

In der Phosgenherstellung in Reaktor **H** verhält sich Kohlenstoffdioxid inert und beeinträchtigt die Reaktion nicht.

Grundsätzlich kann der chlorreiche Produktstrom **P9** auch anderen Anwendungsgebieten neben der Phosgenherstellung zugeführt werden, sofern erhöhte Kohlenstoffdioxidgehalte in dem geplanten Anwendungsgebiet nicht stören.

Das erfindungsgemäße Verfahren kann noch weitere Schritte neben den genannten (i) bis (xi) umfassen (bspw. die Kompression von Gasströmen oder die Reinigung oder Trocknung eines Produktstromes vor dessen Verwendung im nächsten Verfahrensschritt), die zur Vereinfachung der Darstellung oben nicht eigens erwähnt wurden, weil sie den Kern der Erfindung nicht betreffen.

Nachfolgend wird die Erfindung anhand der Figuren 1 bis 4 noch näher erläutert:
Figur 1 zeigt eine vereinfachte, auf die wichtigsten Schritte reduzierte Darstellung des erfindungsgemäßen Verfahrens ohne Kompressionsschritte und ohne Stufen zur Reinigung oder Trocknung von Produktströmen vor deren Verwendung im nächsten Schritt.
Figur 2 zeigt beispielhaft eine mögliche Ausführungsform des erfindungsgemäßen Verfahrens.
Figur 3 zeigt eine weitere mögliche Ausführungsform des erfindungsgemäßen Verfahrens.
Figur 4 zeigt ein Verfahren nach dem Stand der Technik.

Der Übersichtlichkeit halber werden in allen Figuren die gleichen Bezeichnungen für Stoffströme verwendet, z. B. wird das Rohprodukt der Phosgenierung in Reaktor **A** immer als **P1** bezeichnet, auch wenn seine exakte Zusammensetzung je nach Anlagenaufbau variieren kann.

### Figur 1:

Die bei der Isocyanatherstellung in Reaktor **A** anfallende Produktstrom **P1** wird in der Trennvorrichtung **B** in den Isocyanat-haltigen Produktstrom **P2** und den Gasstrom **P3** aufgetrennt. **P3** wird als Chlorwasserstoff-haltiger Rohgasstrom bereitgestellt und enthält neben Chlorwasserstoff unter anderem Anteile an Kohlenstoffdioxid, Kohlenstoffmonoxid und nicht in Reaktor **A** umgesetztes Phosgen.

**P3** wird nach Vermischung mit dem Sauerstoff-haltigen Strom **P4** als **P5** der Deacon-Reaktion **(C)** zugeführt. **C** wird als Kaskade von adiabat betriebenen Reaktoren mit Zwischenkühlung ausgestaltet. Dabei wird der Hauptteil des Chlorwasserstoffs an einem heterogenen Ruthenium-Katalysator zu Chlor und Wasser umgesetzt. In Nebenreaktionen wird das im Gasstrom **P3** enthaltene Kohlenstoffmonoxid und Phosgen zu Kohlenstoffdioxid und Chlor umgesetzt. Der nicht umgesetzte Chlorwasserstoff und der größte Teil des erzeugten Wassers im austretenden Produktstrom **P6** werden in einer Trennvorrichtung **D** als Salzsäure **P7** vom Produktgas **P6** abgetrennt. Dazu wird der Trennvorrichtung **D** zusätzliches frisches Wasser oder verdünnte Salzsäure zugeführt (Strom **E2).**

Der so erhaltene Produktgasstrom **P8** wird in einer sich anschließenden Trennvorrichtung **E** in einen flüssigen, chlorreichen Produktstrom **P9** und einen gasförmigen, chlorarmen Produktstrom **P10** durch Kondensation aufgetrennt. Der flüssige Strom **P9** ist, entsprechend des Partialdrucks von Sauerstoff in der Gasphase, mit gelöstem Sauerstoff gesättigt.

Hinter der Trennvorrichtung **E** wird vom verbleibenden Gasstrom **P10** ein Teilstrom **P11** ausgeschleust. Dieser Teilstrom enthält noch Chlor sowie eine Restmenge an Kohlenstoffdioxid und muss in eine Abgasbehandlungsstufe **F** geführt werden, in der das Chlor mit einem Natronlauge enthaltenden Strom **E3** entfernt wird. Anschließend verlässt der gereinigte Gasstrom **P13** die Anlage. Der Abwasserstrom **P14** enthält das entfernte Chlor in chemisch gebundener Form als Hypochlorit und Chlorid sowie das Kohlenstoffdioxid als Carbonat.

Der nach der Ausschleusung verbleibende Gasstrom **P12** wird mit einem Frisch-Sauerstoffstrom **E4** als Ersatz für den im Prozess verbrauchten Sauerstoff vermischt. Der so erhaltene Strom **P4** wird dann wie oben beschrieben mit dem Chlorwasserstoff-haltigen Gasstrom **P3** vereinigt.

Der Chlor, Kohlenstoffdioxid und gelösten Sauerstoff enthaltende Flüssigkeitsstrom **P9** wird in einen Verdampfer **G** geleitet und nach Zusatz von Kohlenstoffmonoxid **E5** und Chlor **E6** im Reaktor **H** zu Phosgen umgesetzt. Dabei können **E5**, **E6** und der verdampfte Strom **P9** vor Eintritt in den Reaktor **H** oder im Reaktor **H** vermischt werden. Die Figur 1 zeigt nur die letztere Variante. Das zugeführte Chlor **E6** kommt bevorzugt aus einer Chlor-Alkali-Elektrolyse und weist dann einen geringen Sauerstoffanteil von typischerweise 0,1 Vol.-% bis 2 Vol.-% auf. Das zugeführte Kohlenstoffmonoxid **E5** enthält ebenfalls Spuren von Sauerstoff, vor allem, wenn es aus der Reaktion von Koks mit Sauerstoff herrührt. Da das Kohlenstoffmonoxid im stöchiometrischen Überschuss zugeführt wird, wird das Chlor überwiegend bis vollständig umgesetzt. Zusätzlich reagiert überschüssiges Kohlenstoffmonoxid mit den vorhandenen Sauerstoffspuren und bildet Kohlenstoffdioxid.

In der Trennvorrichtung **I** wird der Hauptteil des Phosgens und ein Teil des Kohlenstoffdioxids als Strom **P16** auskondensiert. Der verbleibende gasförmige Strom **P17** wird in eine Abgasbehandlung geführt (nicht gezeichnet), da er noch Phosgen enthält, das vor Abgabe in die Atmosphäre zerstört werden muss. Die Zerstörung erfolgt üblicherweise mit Wasser an Aktivkohle. Mit diesem Gasstrom wird der Hauptteil des Kohlenstoffdioxids aus dem Anlagenverbund aus Deacon-Prozess und Isocyanatanlage entfernt, ohne dass dazu der Einsatz von Natronlauge oder anderen Basen notwendig ist.

Der flüssige Strom **P16** wird zusammen mit dem Amin-haltigen Strom **E1** in den Reaktor **A** geführt und dort zum korrespondierenden Isocyanat umgesetzt.

### Figur 2:

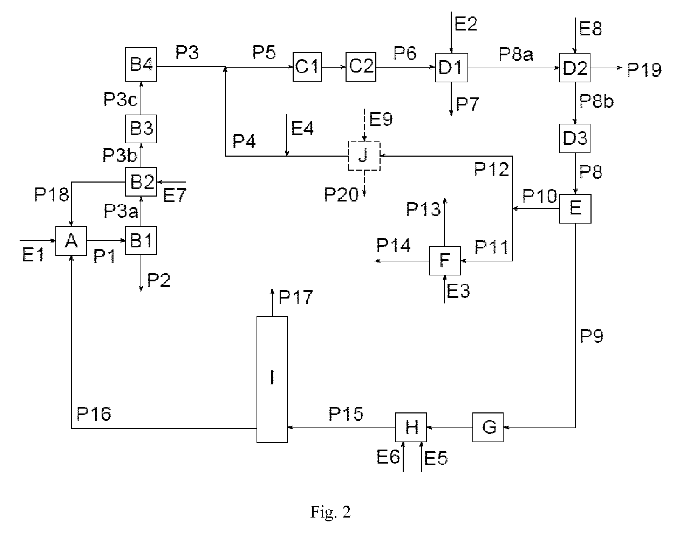

Figur 2 zeigt eine bevorzugte Ausführungsform des erfindungsgemäßen Verfahrens. Sie enthält alle Schritte aus Figur 1 und zeigt darüber hinaus weitere bevorzugte Details:

Die Trennvorrichtung **B** umfasst hier einen Kondensator **B1,** eine Absorberkolonne **B2,** einen Kompressor **B3** und eine Reinigungsstufe **B4.** In **B1** wird der flüssige Strom **P2** sowie ein gasförmiger Strom **P3a** erhalten. **P3a** wird in den unteren Teil der Absorberkolonne **B2** geleitet, an deren Kopf ein organisches Lösungsmittel, bevorzugt das in der Phosgenierung eingesetzte Lösungsmittel, kalt als Strom **E7** zugeführt wird. Aus dem zugeführten Gasstrom **P3a** werden weitere Bestandteile wie überschüssiges Phosgen entfernt und als Strom **P18** in den Reaktor **A** rückgeführt. Das am Kopf der Absorberkolonne **B2** austretende Gas **P3b** wird in einen Kompressor **B3** geführt und dort auf Drücke von 2,0 bar bis 25 bar (abs.) verdichtet.

Der verdichtete Gasstrom **P3c** wird zur weiteren Reinigung vor der Vermischung mit **P4** in eine Reinigungsstufe **B4** (bevorzugt eine Kältefalle und/oder Adsorptionsstufe, z. B. eine Behandlung mit Aktivkohle) geführt. Auf diese Weise wird ein gereinigter gasförmiger Produktstrom **P3** erhalten.

Die oxidative Verfahrensstufe **C** umfasst hier einen Wärmeübertrager **C1,** in dem der Strom **P5** auf die gewünschte Reaktionstemperatur erhitzt wird und eine Kaskade von Reaktoren **C2,** in der die eigentliche Oxidationsreaktion stattfindet.

Die Trennvorrichtung **D** umfasst hier einen Abscheideprozess **D1,** in dem Salzsäure **P7** und ein gasförmiger Strom **P8a** erhalten werden, eine Trocknungsstufe **D2,** in der der Wassergehalt des rohen Produktgases **P8a** durch Kontaktieren mit konzentrierter Schwefelsäure **E8** (bevorzugt in einer Kolonne) verringert und so ein getrockneter Strom **P8b** neben verdünnter Schwefelsäure **P19** erhalten wird, und einen Kompressor **D3,** in dem durch Verdichtung auf Drücke von bevorzugt zwischen 5,0 bar und 30 bar (abs.) von **P8b** der gasförmige Produktstrom **P8** erhalten wird. Der Trocknungsschritt **D2** kann alternativ auch mit Molsieben oder anderen Trocknungsmitteln wie z. B. ionischen Flüssigkeiten erfolgen.

Die Trennvorrichtung **E** umfasst einen Kondensationsschritt, wobei hauptsächlich Chlor flüssig abgetrennt wird. Im Chlor ist neben Sauerstoff hauptsächlich Kohlenstoffdioxid gelöst.

Optional wird in dieser Ausführungsform der Strom **P12** vor seiner Vermischung mit **E4** durch eine Wäsche **J** (gestrichelt gezeichnet) geführt, in der das Gas mit einem Wasserstrom **E9** unter Erhalt eines Abwasserstroms **P20** gewaschen wird.

### Figur 3:

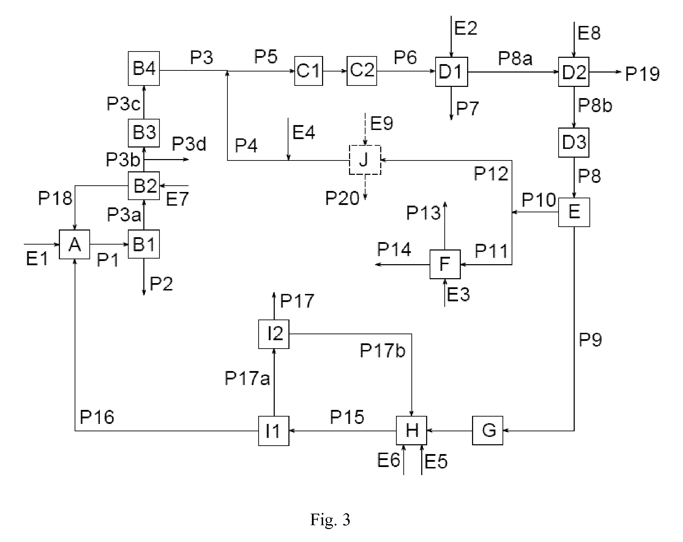

Figur 3 zeigt eine bevorzugte Ausführungsform des erfindungsgemäßen Verfahrens. Sie enthält alle Schritte aus Figur 2 und zeigt darüber hinaus weitere bevorzugte Details:

Von Strom **P3b** wird ein kleiner Teilstrom **P3d** abgezweigt und zu einem Chlorwasserstoff-Absorber geführt (nicht gezeichnet), um wässrige Salzsäure als Nebenprodukt, z. B. für eine Salzsäureelektrolyse, zu erzeugen. Dabei wird auch ein Teil des Kohlenstoffdioxids, Kohlenstoffmonoxids und Phosgens ausgeschleust. Der verbleibende Strom wird im Kompressor **B3** verdichtet. Diese Abzweigung eines Teilstroms der in der Auftrennung von **P1** erhaltenen Gasphase in einen Chlorwasserstoff-Absorber kann auch ohne den Absorptionsschritt in **B2** verwirklicht werden.

Die Trennvorrichtung **I** umfasst hier zwei Stufen, **I1** und **12.** In **I1** werden der flüssige, phosgenreiche Strom **P16** und ein gasförmiger Strom **P17a** erhalten. In **12** wird **P17a** aufgeteilt in den Purge-Strom **P17** und einen in den Reaktor **H** zurückgeführten Strom **P17b.**

### Figur 4:

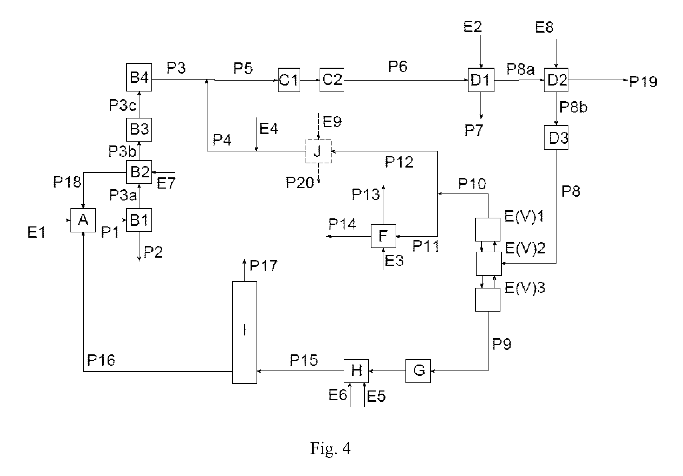

Mit Hilfe von Figur 4 wird die übliche Betriebsweise ohne gemeinsame Kondensation von Chlor und Kohlenstoffdioxid beschrieben. Zur Unterscheidung von den erfindungsgemäßen Figuren sind Apparate in einer nicht erfindungsgemäßen Ausgestaltung durch ein "(V)" (Vergleich) gekennzeichnet. Der grundsätzliche Anlagenaufbau entspricht mit Ausnahme der Trennvorrichtung **E(V)** demjenigen von Figur 2.

Nach dem Stand der Technik wird als Trennvorrichtung **E(V)** eine *Destillationskolonne* eingesetzt. Der Kopfkondensator **E**(**V**)**1** trennt Chlor und Kohlenstoffdioxid als Flüssigkeitsstrom ab. Der Flüssigkeitsstrom rieselt die Stufen (als **E(V)2** angedeutet) der Destillationskolonne herunter und wird im Gegenstrom mit verdampftem Chlor gestrippt. Dabei wird sowohl das Kohlenstoffdioxid als auch gelöster Sauerstoff aus der Flüssigkeit ausgetrieben und es kommt nahezu Kohlenstoffdioxid- und Sauerstoff-freies, flüssiges Chlor im Sumpf der Destillationskolonne an. Ein Teil dieses Stroms wird in einen Sumpfverdampfer **E(V)3** geführt, verdampft und als Strippgas in die Destillationskolonne zurückgegeben. Der Rest wird als Strom **P9** abgezogen. Der gasförmig aus dem Kopfkondensator **E**(**V**)**1** austretende Strom **P10** enthält jetzt das Kohlenstoffdioxid, das der Stufe **E(V)** mit dem Strom **P8** zugeführt wurde. Dies hat zur Folge, dass mit Strom **P9** nur ein kleinerer Anteil des aus dem Anlagenverbund insgesamt auszuschleusenden Kohlenstoffdioxids über den Verdampfer **G** und den Reaktor **H** in die Trennvorrichtung **I** gelangt und mit dem Strom **P17** ausgeschleust wird.

### Beispiele

Die Beispiele wurden mit einem Simulationswerkzeug für stationäre Prozesssimulationen erstellt. Die Randbedingungen der Simulation sind im Folgenden beschrieben. Die Massenströme sind für alle Beispiele so skaliert, dass im Reaktor **A** 100 kg/h Isocyanat produziert werden.

### Beispiel 1 (erfindungsgemäß)

Grundlage der Simulation ist Figur 2.

Mit Strom **P16** wird dem Reaktor **A** Phosgen in einem Überschuss von 100 % der Stöchiometrie gegenüber dem im Strom **E1** enthaltenen primären Amin Toluylendiamin (TDA) zugeführt. Die Umsetzung zum Toluylendiisocyanat (TDI) erfolgt bei 150 °C und einem Druck von 2,6 bar. Der größte Teil des nicht umgesetzten Phosgens wird zusammen mit einem Lösungsmittel (ortho-Dichlorbenzol, ODB), dem TDI sowie dem Nebenprodukt HCl als Strom **P1** abgezogen und in **B1** auf 35 °C abgekühlt. Das Produkt TDI wird aus **B1** mit dem Strom **P2** flüssig abgezogen, HCl, Phosgen und weitere Komponenten verlassen mit dem Strom **P3a** die Stufe **B1** gasförmig. **P3a** wird in der Absorberstufe **B2** mit dem Strom **E7,** der frisches ODB enthält, in Kontakt gebracht. Dabei wird das in **P3a** enthaltene Phosgen größtenteils absorbiert. Die HCl verlässt den Absorber gasförmig mit dem Strom **P3b.** In **P3b** sind unter anderem noch ca. 0,23 Vol.-% Phosgen, ca. 0,58 Vol.-% CO₂ und 0,02 Vol.-% CO, ca. 1,2 Vol.-% N₂ aus Inertisierungen bzw. Druckhaltungen sowie Spuren des Lösungsmittels ODB enthalten. Der Hauptteil des Lösungsmittels und des Phosgens werden im Strom **P18** flüssig abgezogen und in den Reaktor **A** rückgeführt.

Im Kompressor **B3** wird das HCl-Gas auf 6 bar komprimiert und als Strom **P3c** der Reinigungsstufe **B4** zugeführt, in der hauptsächlich das Lösungsmittel ODB durch Adsorption an Aktivkohle entfernt und ein gereinigter HCl-Gasstrom **P3** bereitgestellt wird. Anschließend wird ein sauerstoffhaltiger Rückstrom **P4** zugemischt, so dass sich im Strom **P5** ein molares Verhältnis von HCl/O₂ = 2 einstellt. **P5** wird im Wärmeübertrager **C1** auf 290 °C erhitzt und in einer Kaskade **C2** von sieben adiabat betriebenen Reaktoren mit Zwischenkühlung zu Chlor und Wasser oxidiert. Dabei wird ein HCl-Umsatz von 85 % erzielt. Wegen des Sauerstoff-Überschusses wird das im HCl-Gas enthaltene Phosgen und CO zu CO₂ umgesetzt.

Im Abscheider **D1** wird nicht umgesetztes Hel-Gas und das entstandene Wasser abgetrennt. **D1** besteht aus einer Absorptionskolonne mit zwei übereinander angeordneten Packungssegmenten und darüber liegenden Stoffaustauschböden. Die Packungssegmente werden jeweils über einen separaten Flüssigkeitskreislauf berieselt, auf dem obersten Boden wird ein Wasserstrom **E2** zugeführt. Das Reaktionsgas **P6** aus **C2** wird unter das unterste Packungssegment in den Abscheider **D1** geführt. Um dieses Packungssegment wird ein gekühlter Flüssigkeitskreislauf aus 30%-iger Salzsäure geführt. P6 wird dadurch abgekühlt und der größte Teil des HCl-Gases und des Wassers abgeschieden und dem Sumpf von **D1** als 30%-iger Salzsäurestrom P7 entnommen. Das restliche Gas gelangt in das obere Packungssegment, das mit einer gekühlten, im Kreis geführten 14%-igen Salzsäure berieselt wird. In dieser Salzsäure werden HCl und Wasser aus dem restlichen Gas absorbiert. Das oben aus dieser Packung austretende Gas enthält nun kaum noch HCl und wenig Wasser. Zur fast vollständigen Abtrennung der HCl wird es durch die Böden geführt, die im Gegenstrom mit einem Frischwasserstrom E2 beaufschlagt werden. Dadurch gelingt eine fast vollständige Abtrennung der HCl.

Anschließend wird der von HCl und dem größten Teil des Wassers befreite Gasstrom P8a in einer Trocknungsstufe **D2** entwässert. Dazu wird ein mit Schwefelsäure berieselter Absorber verwendet. Er besteht ähnlich wie **D1** aus einem Packungsteil und darüber angeordneten Böden. Der Gasstrom **P8a** wird unter der Packung zugeführt. Der Packungsteil wird mit einem gekühlten 78%-igen Schwefelsäurekreislauf berieselt, in dem der Hauptteil des verbleibenden Wassers absorbiert und als 78%-iger Schwefelsäurestrom **P19** aus dem Sumpf von **D2** ausgeschleust wird. Das aus der Packung austretende Gas wird durch die Böden geleitet, die im Gegenstrom mit einem 96%-igen Schwefelsäurestrom **E8** beaufschlagt werden, so dass das Wasser bis auf Spuren aus dem Gasstrom entfernt wird.

Der getrocknete Gasstrom **P8b,** der aus **D2** austritt, enthält im Wesentlichen das erzeugte Chlor, den überschüssigen Sauerstoff sowie Kohlendioxid und Stickstoff. Er wird mit dem Kompressor **D3** auf 12 bar verdichtet. Anschließend wird der so erhaltene Strom **P8** in den Kondensator **E** geführt und auf -40°C abgekühlt. Dabei kondensieren 99,8 % des in **C2** erzeugten Chlors und ein Teil des Kohlendioxids. Der in **P8** enthaltene Sauerstoff löst sich in Spuren im Kondensat. Der so entstandene Kondensatstrom **P9** weist ca. 1,15 Massen-% CO₂ und ca. 800 ppm Sauerstoff auf. Der verbleibende Gasstrom **P10** verlässt den Kondensator **E. P10** umfasst den überschüssigen Sauerstoff, den Stickstoff, das restliche, nicht kondensierte Chlor und Kohlendioxid. Zur Abtrennung von Inerten und Nebenkomponenten wird ein Teilstrom **P11** des verbleibenden Gasstroms **P10** ausgeschleust. **P11** wird in die Abgasbehandlungsstufe **F** geleitet und dort mit einem Natronlauge enthaltenden Strom **E3** entchlort. Das in **P11** enthaltene Kohlendioxid wird ebenfalls mit der Natronlauge aus **E3** umgesetzt. Auf diese Weise werden ca. 10 % des insgesamt ausgeschleusten Kohlendioxids an dieser Stelle abgetrennt. Der gereinigte, chlor- und kohlendioxidfreie Gasstrom **P13** und das Abwasser **P14** verlassen **F. P14** enthält das mit der Natronlauge zu Natriumcarbonat umgesetzte Kohlendioxid und das zu NaCl und NaOCl umgewandelte Chlor.

Der nach der Abtrennung des Teilstroms **P11** verbleibende Gasstrom **P12** kann optional in einer Waschstufe **J** mit einem Wasserstrom **E9** gewaschen und wiederbefeuchtet werden.

Überschüssiges Waschwasser verlässt diese Stufe als Strom **P20.** Der gewaschene Gasstrom wird nun mit frischem Sauerstoff **E4** vermischt und dann als sauerstoffhaltiger Rückstrom **P4** mit dem gereinigten HCl-Gasstrom **P3** vermischt.

Der Kondensatstrom **P9** wird im Verdampfer **G** verdampft und in den Reaktor **H** geleitet. Dort wird auch ein zusätzlicher Chlorstrom **E6** eingeleitet, der die Verluste (z.B. im Ausschleusestrom **P11** oder die in **C2** nicht umgesetzte HCl) ausgleicht. **E6** stammt aus einer Chlor-Alkali-Elektrolyse und ist mit 0,2 Vol.-% Sauerstoff verunreinigt. Zur Phosgenherstellung wird **H** weiterhin ein CO-Strom **E5** zugeführt. **E5** enthält als Nebenkomponente ebenfalls Sauerstoff (0,1 Vol.-%). Um bei der Phosgenherstellung alles Chlor umzusetzen, wird CO im 6%-igen stöchiometrischen Überschuss zugeführt. Der in **E5**, **E6** und **P9** enthaltene Sauerstoff wird in einer Nebenreaktion mit einem Teil des CO zu Kohlendioxid umgesetzt. Der Strom **P15** umfasst das in **H** erzeugte Phosgen und wird im Kondensator **I** bei 1,6 bar auf -25°C abgekühlt, wobei 98,8% des in **H** erzeugten Phosgens und ein Teil des Kohlendioxids kondensiert und als Strom **P16** mit ca. 0,51 Gew% CO₂ den Kondensator verlässt. In **P16** sind ebenfalls Spuren von CO gelöst. Die verbleibende Gasphase mit dem restlichen Phosgen, dem CO und weiteren Inerten und Nebenkomponenten wird als Abgasstrom **P17** ausgeschleust. An dieser Stelle wird der Rest des auszuschleusenden Kohlendioxids abgetrennt.

### Beispiel 2 (erfindungsgemäß)

Grundlage der Simulation ist Figur 3.

Beispiel 2 enthält dieselben Schritte wie Beispiel 1, jedoch wird zusätzlich ein Teil des HCl-Gases aus dem Absorber **B2** abgezweigt und zu einem HCl-Absorber geführt (nicht gezeichnet). Weiterhin wird ein Teil des gasförmig den Kondensationsschritt **I** verlassenden, den CO-Überschuss enthaltenden Stroms in den Reaktor **H** zurückgeführt.

Die Stufen **A, B1** und **B2** werden genauso betrieben, wie in Beipiel 1 beschrieben. Die HCl verlässt den Absorber gasförmig mit dem Strom **P3b. P3b** enthält unter anderem noch ca. 0,24 Vol.-% Phosgen, ca. 1,2 Vol.-% N₂ aus Inertisierungen bzw. Druckhaltungen sowie Spuren des Lösungsmittels ODB. Sein Gehalt an CO₂ ist mit 1,5 Vol.-% jetzt jedoch deutlich höher als in Beispiel 1, da durch die Rückführung im Kondensationsschritt **11/12** die dort ausgeschleuste Menge kleiner geworden ist und es so zu einer Aufkonzentrierung kommt. CO kommt dagegen nur noch in Spuren vor. Von **P3b** werden 10 % als Strom **P3d** ausgeschleust und zu einem Hel-Absorber geführt (nicht gezeichnet). Dadurch wird ein Teil des in **P3b** enthaltenen Phosgens und des N₂ sowie ca. 30 % des insgesamt auszuschleusenden CO₂ abgetrennt.

Der verbleibende Gasstrom wird im Kompressor **B3** auf 6 bar komprimiert und als Strom **P3c** in die Reinigungsstufe **B4** geführt.

Von Strom **P3c** wird in der Reinigungsstufe **B4** hauptsächlich das Lösungsmittel ODB durch Adsorption an Aktivkohle entfernt und ein gereinigter HCl-Gasstrom **P3** bereitgestellt. Anschließend wird ein sauerstoffhaltiger Rückstrom **P4** zugemischt, so dass sich im Strom **P5** ein molares Verhältnis von HCl/O₂ = 2 einstellt.

Die Stufen **C1, C2, D1, D2, D3** und **E** werden ebenfalls so betrieben, wie in Beipiel 1 beschrieben. In **P9** werden wieder 99,8% des es in **C2** erzeugten Chlors kondensiert. Da in **P3c** aber die CO₂ Konzentration schon höher liegt, weist **P9** auch einen höheren CO₂-Gehalt auf als in Beispiel 1, nämlich 2,4 Massen-%. Der Sauerstoffgehalt beträgt 740 ppm.

Der verbleibende Gasstrom **P10** verlässt den Kondensator **E** und wird wiederum wie in Beispiel 1 teilweise ausgeschleust. Durch diese Ausschleusung werden jetzt ca. 20 % des auszuschleusenden CO₂ abgetrennt, da auch die Ausschleusung eine höhere CO₂-Konzentration aufweist. Die Behandlung der Ausschleusung in **F** und des verbleibenden Gasstroms in der optionalen Stufe **J** sowie dessen anschließende Vermischung mit frischem Sauerstoff **E4** und Rückführung als sauerstoffhaltiger Strom **P4** in den gereinigten HCl-Gasstrom **P3** erfolgt wie in Beispiel 1 beschrieben.

Der Kondensatstrom **P9** wird wie in Beispiel 1 im Verdampfer **G** verdampft und in den Reaktor **H** geleitet. Der zusätzliche Chlorstrom **E6** ist jetzt größer als in Beispiel 1, da zusätzlich zu den Verlusten im Ausschleusestrom **P11** und der in **C2** nicht umgesetzten HCl auch die HCl-Ausschleusung **P3d** kompensiert werden muss. **E6** stammt aus einer Chlor-Alkali-Elektrolyse und ist mit 0,2 Vol.-% Sauerstoff verunreinigt. Zur Phosgenherstellung wird **H** wiederum der CO-Strom **E5** mit 0,1 Vol.-% Sauerstoff als Nebenkomponente zugeführt. Um bei der Phosgenherstellung alles Chlor umzusetzen, liegt CO im 6%-igen stöchiometrischen Überschuss am Eingang von Reaktor **H** vor. Da ein Teil des hinter dem Reaktor **H** in der Kondensationsstufe **I1** abgetrennten Gasstroms **P17a** zurückgeführt wird, muss mit **E5** nur noch ca. 95,4 % des benötigten CO bereitgestellt werden. Damit ist der zugeführte CO-Strom **E5** kleiner als in Beispiel 1. Der in **E5**, **E6** und **P9** enthaltene Sauerstoff wird in einer Nebenreaktion mit einem Teil des CO zu Kohlendioxid umgesetzt. Der Strom **P15** umfasst das in **H** erzeugte Phosgen und wird im Kondensator **I1** bei 1,6 bar auf -25°C abgekühlt, wobei 99,8 % des in **H** erzeugten Phosgens und ein Teil des Kohlendioxids kondensiert und als Strom **P16** mit ca. 1,3 Massen-% CO₂ den Kondensator verlässt. Der CO₂-Anteil ist höher als in Beispiel 1, weil mit der Rückführung in den Reaktor **H** ebenfalls CO₂ rückgeführt wird und sich so aufkonzentriert. In **P16** sind Spuren von CO gelöst. Die verbleibende Gasphase mit dem restlichen Phosgen, dem CO und weiteren Inerten und Nebenkomponenten wird als Gasstrom **P17a** abgezogen. **P17a** wird in **12** aufgeteilt, 10% werden als Strom **P17** ausgeschleust, der Rest wird als Strom **P17b** in den Reaktor **H** rückgeführt. Auf diese Weise wird hier der Rest des auszuschleusenden Kohlendioxids abgetrennt.

### Beispiel 3 (Vergleichsbeispiel)

Grundlage der Simulation ist Figur 4.

Beispiel 3 enthält dieselben Schritte wie Beispiel 1, jedoch ist **E** jetzt als Destillationskolonne ausgeführt, um das abgetrennte flüssige Chlor von CO₂ und gelöstem Sauerstoff zu befreien.

Die Stufen **A, B1**, **B2, B3** und **B4** werden genauso betrieben, wie in Beipiel 1 beschrieben. Die HCl verlässt den Absorber **B2** gasförmig mit dem Strom **P3b. P3b** enthält unter anderem noch ca. 0,22 Vol% Phosgen, ca. 1,2 Vol.-% N₂ aus Inertisierungen bzw. Druckhaltungen sowie Spuren des Lösungsmittels ODB. Sein Gehalt an CO₂ und CO ist mit 0,15 Vol.-% (CO₂) und 0,03 Vol.-% (CO) sehr viel niedriger als in Beispiel 1, da durch die Destillation des flüssigen Chlors mit dem Strom **P9** im Wesentlichen kein CO₂ und kein Sauerstoff in den Phosgen-Herstellungsschritt **H** geleitet wird.

Der Gasstrom **P3b** wird im Kompressor **B3** auf 6 bar komprimiert und als Strom **P3c** in die Reinigungsstufe **B4** geführt.

Von Strom **P3c** wird in der Reinigungsstufe **B4** hauptsächlich das Lösungsmittel ODB durch Adsorption an Aktivkohle entfernt und ein gereinigter HCl-Gasstrom **P3** bereitgestellt. Anschließend wird ein sauerstoffhaltiger Rückstrom **P4** zugemischt, so dass sich im Strom **P5** ein molares Verhältnis von HCl/O₂ = 2 einstellt.

Die Stufen **C1**, **C2, D1, D2** und **D3** werden ebenfalls so betrieben, wie in Beipiel 1 beschrieben.

Die Stufe **E** ist jetzt als Destillationskolonne ausgebildet. Sie besteht aus einem Kopfkondensator **E**(**V**)**1**, den Destillationsstufen **E(V)2,** auf die der Strom **P8** geführt wird, und dem Sumpfverdampfer **E(V)3.** Im Kopfkondensator **E**(**V**)**1** wird auf -40°C abgekühlt, der Sumpfverdampfer **E(V)3** verdampft ca. 1/3 der Menge des zugeführten Stroms **P8.** Damit ergibt sich ein Sumpfstrom **P9,** in dem 99,7 % des in **C2** erzeugten Chlors abgetrennt werden. In **P9** sind nur noch 0,17 Massen-% CO₂ und kein Sauerstoff mehr enthalten.

Der verbleibende Gasstrom **P10** verlässt die Destillationskolonne **E** am Kopfkondensator **E**(**V**)**1** und wird wiederum wie in Beispiel 1 teilweise ausgeschleust. Dadurch werden jetzt ca. 70 % des auszuschleusenden CO₂ abgetrennt, da durch die Destillation kaum CO₂ mit dem Flüssigchlor abgetrennt wird und es sich daher in der Rückführung anreichert.

Die Behandlung der Ausschleusung in **F** und des verbleibenden Gasstroms in der optionalen Stufe **J** sowie dessen anschließende Vermischung mit frischem Sauerstoff **E4** und Rückführung als sauerstoffhaltiger Strom **P4** in den gereinigten HCl-Gasstrom **P3** erfolgt wie in Beispiel 1 beschrieben.

Der Kondensatstrom **P9** wird wie in Beispiel 1 im Verdampfer **G** verdampft und in den Reaktor **H** geleitet. Der zusätzliche Chlorstrom **E6** stammt aus einer Chlor-Alkali-Elektrolyse und ist mit 0,2 Vol.-% Sauerstoff verunreinigt. Zur Phosgenherstellung wird **H** wiederum der CO-Strom **E5** mit 0,1 Vol.-% Sauerstoff als Nebenkomponente zugeführt. Um bei der Phosgenherstellung alles Chlor umzusetzen, liegt CO im 6%-igen stöchiometrischen Überschuss am Eingang von Reaktor **H** vor.

Der in **E5** und **E6** enthaltene Sauerstoff wird in einer Nebenreaktion mit einem Teil des CO zu Kohlendioxid umgesetzt. Der Strom **P15** umfasst das in **H** erzeugte Phosgen und wird im Kondensator **I1** bei 1,6 bar auf -25°C abgekühlt, wobei 99 % des in **H** erzeugten Phosgens und ein Teil des Kohlendioxids kondensiert und als Strom **P16** mit ca. 0,13 Massen-% CO₂ den Kondensator verlässt. In **P16** sind ebenfalls Spuren von CO gelöst. Die verbleibende Gasphase mit dem restlichen Phosgen, dem CO und weiteren Inerten und Nebenkomponenten wird als Gasstrom **P17** ausgeschleust. Auf diese Weise wird hier der Rest des auszuschleusenden Kohlendioxids abgetrennt. Die folgende Übersicht stellt die wesentlichen Simulationsergebnisse einander gegenüber:

| | **Beispiel 1 (****Figur 2****)** | **Beispiel 2 (****Figur 3****)** | **Beispiel 3 (****Figur 4****)** |
|---|---|---|---|
| Anteil der CO₂-Ausschleusung über die Abgasbehandlung **F** (Strom **P11**) an der gesamten CO₂ Ausschleusung | 9% | 18% | 70% |
| Anteil der CO₂-Ausschleusung über die Trennvorrichtung **I** (Strom **P17**) an der gesamten CO₂-Ausschleusung | 91 % | 52% | 30% |
| Anteil der CO₂-Ausschleusung über ausgeschleustes HCl-Gas (Strom **P3d**) an der gesamten CO₂ Ausschleusung | - | 30% | - |
| NaOH-Verbrauch (Strom **E3**) in der Abgasbehandlung **F** im Vergleich zu Beispiel 3 | 33 % | 41% | 100% |
| Kälteleistung in der Trennvorrichtung **E** zur Kondensation des Chlors im Vergleich zu Beispiel 3 | 74% | 70% | 100% |

Wie der Übersicht zu entnehmen ist, wird mit dem erfindungsgemäßen Verfahren der NaOH-Verbrauch erheblich reduziert. Außerdem wird auch weniger Kälteleistung benötigt um das Chlor abzutrennen, da in der Destillationskolonne des Beispiels 3 zusätzlich das im Sumpf mit dem ausgetriebenen CO₂ verdampfte Chlor kondensiert werden muss.

## Patentansprüche

1. Verfahren zur Herstellung eines Isocyanats umfassend die Schritte
(i) Umsetzung eines ein primäres Amin enthaltenden Stroms **E1** mit einem phosgenhaltigen Strom **P16** unter Ausbildung eines Produktstroms **P1**, der das gewünschte Isocyanat, Chlorwasserstoff, nicht umgesetztes Phosgen und Kohlenstoffoxide umfasst,
(ii) Auftrennung des Produktstromes **P1** in einen das Isocyanat umfassenden flüssigen Produktstrom **P2** und einen gasförmigen, Chlorwasserstoff, nicht umgesetztes Phosgen und Kohlenstoffoxide umfassenden Produktstrom **P3,**
(iii) Vermischung des Chlorwasserstoff, nicht umgesetztes Phosgen und Kohlenstoffoxide umfassenden Produktstroms **P3** mit einem gasförmigen, Sauerstoff umfassenden Produktstrom **P4** unter Bildung eines gasförmigen Mischstromes **P5**,
(iv) Oxidation des gasförmigen Mischstromes **P5** an einem Katalysator unter Bildung eines gasförmigen Produktstromes **P6,** der Chlorwasserstoff, Kohlenstoffdioxid, überschüssigen Sauerstoff, Chlor und Wasser umfasst,
(v) Teilweise bis vollständige Abtrennung des Chlorwasserstoffs und des Wassers aus **P6** als Salzsäure-haltigen Strom **P7** unter Ausbildung eines an Chlorwasserstoff und Wasser abgereicherten Produktstromes **P8,**
(vi) Auftrennung des Produktstromes **P8** in einen flüssigen chlorreichen Produktstrom **P9,** der Chlor, Kohlenstoffdioxid und Sauerstoff umfasst, und in einen gasförmigen, chlorarmen Produktstrom **P10,** der die Restmenge des nicht in **P9** enthaltenen Chlors aus **P8** sowie Kohlenstoffdioxid und Sauerstoff umfasst,
(vii) Aufteilung des gasförmigen Produktstromes **P10** in einen gasförmigen Purge-Strom **P11** und in einen gasförmigen Produktstrom **P12,**
(viii) Behandlung des Purge-Stromes **P11** mit einer wässrigen Base **E3** unter Bildung eines gasförmigen Purge-Stromes **P13** und eines flüssigen Abwasserstromes **P14,**
(ix) Vermischung des gasförmigen Stromes **P12** mit einem gasförmigen, Sauerstoff umfassenden Strom **E4** unter Ausbildung des gasförmigen, Sauerstoff umfassenden Produktstromes **P4,** welcher in Schritt (iii) eingesetzt wird,
(x) Verdampfung des flüssigen chlorreichen Produktstroms **P9,** Vermischung des so erhaltenen gasförmigen chlorreichen Produktstroms mit Kohlenstoffmonoxid **E5** und mit Chlor **E6** und Umsetzung des so erhaltenen Gemisches zu einem gasförmigen Produktstrom **P15** umfassend Phosgen und Kohlenstoffoxide,
(xi) Auftrennung des gasförmigen Produktstroms **P15** in einer Trennvorrichtung **I** in einen phosgenreichen Produktstrom **P16,** welcher in Schritt (i) eingesetzt wird und Phosgen und Kohlenstoffoxide umfasst, und in einen phosgenarmen Purge-Strom **P17,** der die Restmenge des nicht in **P16** enthaltenen Phosgens aus **P15** sowie Kohlenstoffoxide umfasst,
wobei
in Schritt (vi) die Auftrennung des Produktstromes **P8** in einen flüssigen chlorreichen Produktstrom **P9** und in einen gasförmigen chlorarmen Produktstrom **P10** durch Abkühlung von **P8** auf eine Temperatur von -10 °C bis -80 °C unter einem absoluten Druck von 1 bar bis 30 bar ohne sich anschließende Destillation erfolgt.

2. Verfahren nach Anspruch 1, bei dem
in Schritt (xi) 35 % bis 99 % des insgesamt auszuschleusenden Kohlenstoffdioxids über den Purge-Strom **P17** ausgeschleust werden.

3. Verfahren nach einem der Ansprüche 1 bis 2, bei dem
in Schritt (ii) **P1** in den flüssigen das Isocyanat umfassenden Produktstrom **P2** und einen gasförmigen Strom aufgeteilt wird, wobei ein Teil des so erhaltenen gasförmigen Stroms ausgeschleust und in einen Chlorwasserstoff-Absorber geleitet wird, und der verbleibende gasförmige Strom als Strom **P3** in Schritt (iii) weiter behandelt wird.

4. Verfahren nach einem der Ansprüche 1 bis 3, bei dem
in Schritt (v) die teilweise bis vollständige Abtrennung des Chlorwasserstoffs und des Wassers aus **P6** durch Behandlung von **P6** mit einem Absorptionsmittel ausgewählt aus Wasser oder 1 bis 20%ige Salzsäure verwirklicht wird.

5. Verfahren nach einem der Ansprüche 1 bis 4, bei dem
in Schritt (vii) die Aufteilung des gasförmigen Produktstromes **P10** in einen gasförmigen Purge-Strom **P11** und in einen gasförmigen Produktstrom **P12** in einem zeitgemittelten Massenverhältnis **P12 : P11** von 100 : 1 bis 5 : 1 erfolgt.

6. Verfahren nach einem der Ansprüche 1 bis 5, bei dem
in Schritt (viii) als wässrige Base **E3** eine wässrige Alkalimetall- oder Erdalkalimetallhydroxidlösung verwendet wird.

7. Verfahren nach einem der Ansprüche 1 bis 6, bei dem
der gasförmige Produktstrom **P12** durch eine Wäsche geführt wird, in der das Gas gewaschen und erst danach in Schritt (ix) mit einem gasförmigen, Sauerstoff umfassenden Strom **E4** vermischt wird.

## Claims

1. A process for preparing an isocyanate comprising the steps of
(i) reacting a stream **E1** comprising a primary amine with a phosgene-comprising stream **P16** to form a product stream **P1,** which comprises said isocyanate, hydrogen chloride, unreacted phosgene, and carbon oxides;
(ii) separating the product stream **P1** into a liquid product stream **P2** comprising said isocyanate and a gaseous product stream **P3** comprising hydrogen chloride, unreacted phosgene, and carbon oxides;
(iii) mixing the product stream **P3** comprising hydrogen chloride, unreacted phosgene, and carbon oxides with a gaseous product stream **P4** comprising oxygen to form a gaseous mixed stream **P5;**
(iv) oxidizing the gaseous mixed stream **P5** on a catalyst to form a gaseous product stream **P6** comprising hydrogen chloride, carbon dioxide, excess oxygen, chlorine, and water;
(v) partially to completely removing the hydrogen chloride and water from **P6** as a stream **P7** comprising hydrochloric acid to form a product stream **P8** depleted in hydrogen chloride and water;
(vi) separating the product stream **P8** into a liquid chlorine-rich product stream **P9,** which comprises chlorine, carbon dioxide, and oxygen, and a gaseous, low-chlorine product stream **P10,** which comprises the residual amount of chlorine from **P8** not contained in **P9,** carbon dioxide, and oxygen;
(vii) dividing the gaseous product stream **P10** into a gaseous purge stream **P11** and a gaseous product stream **P12;**
(viii) treating the purge stream **P11** with an aqueous base **E3** to form a gaseous purge stream **P13** and a liquid waste water stream **P14;**
(ix) mixing the gaseous stream **P12** with a gaseous stream **E4** comprising oxygen to form the gaseous product stream **P4** which comprises oxygen and is employed in step (iii);
(x) evaporating the liquid chlorine-rich product stream **P9,** mixing the gaseous chlorine-rich product stream obtained in this way with carbon monoxide **E5** and with chlorine **E6,** and reacting said mixture obtained in this way to give a gaseous product stream **P15** comprising phosgene and carbon oxides;
(xi) separating the gaseous product stream **P15** in a separating device **I** into a phosgene-rich product stream **P16,** which is employed in step (i) and comprises phosgene and carbon oxides, and into a low-phosgene purge stream **P17** which comprises the residual amount of the phosgene from **P15** which is not contained in **P16** and carbon oxides;
wherein
in step (vi) the separation of the product stream **P8** into a liquid chlorine-rich product stream **P9** and into a gaseous low-chlorine product stream **P10** is carried out by cooling **P8** to a temperature of from -10 °C to -80 °C under an absolute pressure of from 1 bar to 30 bar without subsequent distillation.

2. The process of claim 1, wherein, in step (xi), from 35 % to 99 % of the total carbon dioxide to be sluiced out is sluiced out via the purge stream **P17.**

3. The process of either of claims 1 and 2, wherein, in step (ii), **P1** is divided into the liquid product stream **P2** comprising said isocyanate and a gaseous stream, wherein a portion of said gaseous stream is sluiced out and passed into a hydrogen chloride absorber, and wherein the remaining portion of said gaseous stream is treated further as stream **P3** in step (iii).

4. The process of any of claims 1 to 3, wherein, in step (v), the partial to complete removal of the hydrogen chloride and of the water from **P6** is realized by treatment of **P6** with an absorption agent chosen from water or 1 to 20 % strength hydrochloric acid.

5. The process of any of claims 1 to 4, wherein, in step (vii), the gaseous product stream **P10** is divided into a gaseous purge stream **P11** and into a gaseous product stream **P12** in a weight ratio of **P12 : P11** averaged over time of from 100 : 1 to 5 : 1.

6. The process of any of claims 1 to 5, wherein, in step (viii), an aqueous alkali metal or alkaline earth metal hydroxide solution is used as the aqueous base **E3.**

7. The process of any of claims 1 to 6, wherein the gaseous product stream **P12** is led through a wash in which the gas is washed and only thereafter is mixed in step (ix) with a gaseous stream **E4** comprising oxygen.

## Revendications

1. Procédé de fabrication d'un isocyanate, comprenant les étapes suivantes :
(i) la mise en réaction d'un courant E1 contenant une amine primaire avec un courant P16 contenant du phosgène pour former un courant de produits P1, qui comprend l'isocyanate souhaité, du chlorure d'hydrogène, du phosgène non réagi et des oxydes de carbone,
(ii) la séparation du courant de produits P1 en un courant de produits liquide P2 comprenant l'isocyanate et un courant de produits gazeux P3 comprenant du chlorure d'hydrogène, du phosgène non réagi et des oxydes de carbone,
(iii) le mélange du courant de produits P3 comprenant du chlorure d'hydrogène, du phosgène non réagi et des oxydes de carbone avec un courant de produits gazeux P4 comprenant de l'oxygène pour former un courant de mélange gazeux P5,
(iv) l'oxydation du courant de mélange gazeux P5 sur un catalyseur pour former un courant de produits gazeux P6, qui comprend du chlorure d'hydrogène, du dioxyde de carbone, de l'oxygène en excès, du chlore et de l'eau,
(v) la séparation partielle à totale du chlorure d'hydrogène et de l'eau de P6 sous la forme d'un courant P7 contenant de l'acide chlorhydrique pour former un courant de produits P8 appauvri en chlorure d'hydrogène et en eau,
(vi) la séparation du courant de produits P8 en un courant de produits liquide P9 riche en chlore, qui comprend du chlore, du dioxyde de carbone et de l'oxygène, et en un courant de produits gazeux P10 pauvre en chlore, qui comprend la quantité résiduelle du chlore de P8 non contenu dans P9, ainsi que du dioxyde de carbone et de l'oxygène,
(vii) la division du courant de produits gazeux P10 en un courant de purge gazeux P11 et un courant de produits gazeux P12,
(viii) le traitement du courant de purge P11 avec une base aqueuse E3 pour former un courant de purge gazeux P13 et un courant d'eau résiduaire liquide P14,
(ix) le mélange du courant gazeux P12 avec un courant gazeux E4 comprenant de l'oxygène pour former le courant de produits gazeux P4 comprenant de l'oxygène, qui est utilisé à l'étape (iii),
(x) l'évaporation du courant de produits liquide P9 riche en chlore, le mélange du courant de produits gazeux riche en chlore ainsi obtenu avec du monoxyde de carbone E5 et avec du chlore E6, et la mise en réaction du mélange ainsi obtenu pour former un courant de produits gazeux P15 comprenant du phosgène et des oxydes de carbone,
(xi) la séparation du courant de produits gazeux P15 dans un dispositif de séparation I en un courant de produits P16 riche en phosgène, qui est utilisé à l'étape (i), et comprend du phosgène et des oxydes de carbone, et en un courant de purge P17 pauvre en phosgène, qui comprend la quantité résiduelle du phosgène de P15 non contenu dans P16, ainsi que des oxydes de carbone,
à l'étape (vi), la séparation du courant de produits P8 en un courant de produits liquide P9 riche en chlore et en un courant de produits gazeux P10 pauvre en chlore ayant lieu par refroidissement de P8 à une température de -10 °C à -80 °C à une pression absolue de 1 bar à 30 bar sans distillation ultérieure.

2. Procédé selon la revendication 1, selon lequel à l'étape (xi), 35% à 99% du dioxyde de carbone à évacuer au total est évacué par le courant de purge P17.

3. Procédé selon l'une quelconque des revendications 1 à 2, selon lequel à l'étape (ii), P1 est divisé en le courant de produits liquide P2 comprenant l'isocyanate et en un courant gazeux, une partie du courant gazeux ainsi obtenu étant évacuée et conduite dans un absorbeur de chlorure d'hydrogène, et le courant gazeux restant étant traité ultérieurement en tant que courant P3 dans l'étape (iii).

4. Procédé selon l'une quelconque des revendications 1 à 3, selon lequel à l'étape (v), la séparation partielle à totale du chlorure d'hydrogène et de l'eau de P6 est réalisée par traitement de P6 avec un agent d'absorption choisi parmi l'eau ou l'acide chlorhydrique à 1 à 20 %.

5. Procédé selon l'une quelconque des revendications 1 à 4, selon lequel à l'étape (vii), la division du courant de produits gazeux P10 en un courant de purge gazeux P11 et en un courant de produits gazeux P12 a lieu en un rapport en masse moyenné par le temps P12:P11 de 100:1 à 5:1.

6. Procédé selon l'une quelconque des revendications 1 à 5, selon lequel à l'étape (viii), une solution aqueuse d'hydroxyde de métal alcalin ou de métal alcalino-terreux est utilisée en tant que base aqueuse E3.

7. Procédé selon l'une quelconque des revendications 1 à 6, selon lequel le courant de produits gazeux P12 est conduit dans un lavage, dans lequel le gaz est lavé et n'est mélangé qu'après dans l'étape (ix) avec un courant gazeux E4 comprenant de l'oxygène.
